# FASCICULE DE BREVET EUROPEEN

(11) **EP 2 781 556 B1**
(45) Date de publication et mention de la délivrance du brevet: **01.03.2017**
(21) Numéro de dépôt: 14167009.1
(22) Date de dépôt: 23.03.2007
(51) Int. Cl.: C09B 23/14, C09B 49/12, A61Q 5/08

(54) **Procédé de coloration et d'éclaircissement des matières kératiniques en présence d'un agent réducteur comprenant un colorant disulfure fluorescent**
Färbe- und Aufhellungsverfahren von keratinhaltigen Stoffen in Gegenwart eines Reduktionsmittels, das einen fluoreszierenden Disulfidfarbstoff enthält
Method of colouring and lightening keratin materials in the presence of a reducing agent comprising a fluorescent disulphide dye

(30) Priorité: 24.03.2006 FR 0651035; 19.04.2006 US 792941 P
(43) Date de publication de la demande: 24.09.2014
(62) Demande divisionnaire de: 07731820.2
(73) Titulaire: L'Oréal, 75008 Paris (FR)
(72) Inventeur: Daubresse, Nicolas, 78170 La Celle Saint Cloud (FR); Greaves, Andrew, 77700 Magny-Le-Hongre (FR); Samain, Henri, 91570 Bievres (FR)
(74) Mandataire: Rivière, François Armand

(56) Documents cités:
- WO-A2-2004/091473
- WO-A2-2005/097051
- US-A- 2 904 385
- GEOFFREY J. ASHWELL, ABDUL MOHIB AND JAMES R. MILLER: "Induced rectification from self-assembled monolayers of sterically hindered -bridged chromophores", JOURNAL OF MATERIALS CHEMISTRY, vol. 15, no. 11, 27 janvier 2005 (2005-01-27), pages 1160-1166, XP002419175,
- GEOFFREY J. ASHWELL AND ABDUL MOHIB: "Improved Molecular Rectification from Self-Assembled Monolayers of a Sterically Hindered Dye", J. OF THE AMERICAN CHEMICAL SOCIETY, vol. 127, no. 46, 11 janvier 2005 (2005-01-11), pages 16238-16244, XP002419176,
- GEOFFREY J. ASHWELL, WAYNE D. TYRRELL AND ANNE J. WHITTAM: "Molecular rectification: self-assembled monolayers of a donor?(-bridge)?acceptor chromophore connected via a truncated Au?S?(CH2)3 bridge", JOURNAL OF MATERIALS CHEMISTRY, vol. 13, no. 12, 3 novembre 2003 (2003-11-03), pages 2855-2857, XP002419177,
- TSUBOI K ET AL: "Formation of Merocyanine Self-Assembled Monolayer and Its Nonlinear Optical Properties Probed by Second-Harmonic Generation and Surface Plasmon Resonance", JAPANESE JOURNAL OF APPLIED PHYSICS, JAPAN SOCIETY OF APPLIED PHYSICS, TOKYO, JP, vol. 42, no. 2A, février 2003 (2003-02), pages 607-613, XP002355458, ISSN: 0021-4922
- NARAOKAA R ET AL: "Nonlinear optical property of hemicyanine self-assembled monolayers on gold and its adsorption kinetics probed by optical second-harmonic generation and surface plasmon resonance spectroscopy", CHEMICAL PHYSICS LETTERS, NORTH-HOLLAND, AMSTERDAM, NL, vol. 362, no. 1-2, 13 août 2002 (2002-08-13), pages 26-30, XP002355459, ISSN: 0009-2614
- OKAWA H ET AL: "SYNTHESIS AND CHARACTERIZATION OF AN ALKANETHIOL THIN FILM CONTAINING A HEMICYANINE DYE", MOLECULAR CRYSTALS AND LIQUID CRYSTALS SCIENCE AND TECHNOLOGY. SECTION A. MOLECULAR CRYSTALS AND LIQUID CRYSTALS, GORDON AND BREACH PUBLISHERS, CH, vol. 377, 2002, pages 137-140, XP008056025, ISSN: 1058-725X
- KAJIKAWA K ET AL: "PREPARATION AND OPTICAL CHARACTERIZATION OF HEMICYANINE SELF-ASSEMBLED MONOLAYER ON AU SUBSTRATE", MOLECULAR CRYSTALS AND LIQUID CRYSTALS SCIENCE AND TECHNOLOGY. SECTION A. MOLECULAR CRYSTALS AND LIQUID CRYSTALS, GORDON AND BREACH PUBLISHERS, CH, vol. 370, 2001, pages 277-283, XP008056026, ISSN: 1058-725X
- TSUBOI K ET AL: "REFLECTION SPECTROSCOPY OF MEROCYANINE SELF-ASSEMBLED MONOLAYER ON A GOLD SUBSTRATE", MOLECULAR CRYSTALS AND LIQUID CRYSTALS SCIENCE AND TECHNOLOGY. SECTION A. MOLECULAR CRYSTALS AND LIQUID CRYSTALS, GORDON AND BREACH PUBLISHERS, CH, CH, vol. 322, 1998, pages 191-196, XP008056027, ISSN: 1058-725X
- FUJITA K ET AL: "Solvatochromism of a Merocyanine Derivative in a Self-Assembled Monolayer on Gold Substrates", LANGMUIR, AMERICAN CHEMICAL SOCIETY, NEW YORK, NY, US, vol. 14, no. 26, 3 décembre 1998 (1998-12-03), pages 7456-7462, XP002355460, ISSN: 0743-7463

## Description

L'invention a pour objet un colorant disulfure fluorescent pour la coloration et l'éclaircissement des matières kératiniques.

Il est connu de teindre les fibres kératiniques, notamment humaines, par une coloration directe. Le procédé classiquement utilisé en coloration directe consiste à appliquer sur les fibres kératiniques des colorants directs qui sont des molécules colorées et colorantes ayant une affinité pour les fibres, à les laisser diffuser, puis à rincer les fibres.

Les colorants directs qui sont classiquement utilisés sont par exemple des colorants du type nitrés benzéniques, des colorants anthraquinoniques, des nitropyridines, des colorants du type azoïque, xanthénique, acridinique, azinique ou triarylméthane.

Les colorations qui résultent de l'utilisation de colorants directs sont des colorations temporaires ou semi-permanentes car la nature des interactions qui lient les colorants directs à la fibre kératinique, et leur désorption de la surface et/ou du coeur de la fibre sont responsables de leur faible puissance tinctoriale et de leur mauvaise tenue aux lavages ou à la transpiration.

Par ailleurs, la coloration des fibres kératiniques à partir de colorants directs classiques ne permet pas d'éclaircir de façon notable les fibres kératiniques.

L'éclaircissement de la couleur de fibres kératiniques, plus particulièrement foncées vers des nuances plus claires, en modifiant éventuellement la nuance de celles-ci, constitue une demande importante.

Classiquement, pour obtenir une coloration plus claire on met en oeuvre un procédé de décoloration chimique. Ce procédé consiste à traiter les matières kératiniques telles que les fibres kératiniques, notamment les cheveux, par un système oxydant fort, généralement constitué par du peroxyde d'hydrogène associé ou non à des persels, le plus souvent en milieu alcalin.

Ce système de décoloration présente l'inconvénient de dégrader les fibres et d'altérer leurs propriétés cosmétiques. Les fibres ont en effet tendance à devenir rêches, plus difficilement démêlables et plus fragiles. Enfin, l'éclaircissement ou la décoloration de fibres kératiniques à partir d'agent oxydant est incompatible avec les traitements de modification de la forme desdites fibres particulièrement dans les traitements de défrisage.

Une autre technique d'éclaircissement consiste à appliquer sur les cheveux foncés des colorants directs fluorescents. Cette technique décrite notamment dans le document FR 2830189 permet de respecter la qualité de la fibre kératinique lors du traitement mais les colorants fluorescents employés ne présentent pas une résistance aux shampoings satisfaisante.

Pour augmenter la ténacité des colorations directes, il est connu de fixer des colorants directs par liaison covalente au cheveu. Par exemple, il est connu de faire réagir des colorants à groupements réactifs avec les résidus de cystine ou de cystéine très nombreux dans les fibres kératiniques. Sont ainsi décrits certains colorants portant des fonctions sels de Bunte et isothiuroniums, ou d'autres groupements protecteurs de thiols. Cependant, l'obtention de la forme réactive du colorant nécessite en général l'utilisation de milieux fortement basiques. De plus, les fonctions thiols sont généralement générées en excès ce qui rend nécessaire une étape de post neutralisation à la suite de la coloration.

D'autres colorants disulfures connus pour la coloration des fibres kératiniques sont des dérivés disulfures de dérivés d'aminothiophénol. De tels colorants sont décrits par exemple dans le brevet FR 1156407. Ces colorants peuvent être utilisés dans des conditions relativement douces, en présence d'un milieu légèrement réducteur ou après un pré-traitement réducteur du cheveu. Cependant, ces colorants peuvent occasionner des virages de couleur lors de l'application.

Enfin, le document WO 2005/097051 décrit des colorants disulfures aza-imidazoliums pour la coloration directe de fibres kératiniques.

Le but de la présente invention est de fournir de nouveaux systèmes de coloration avec effet éclaircissant de matières kératiniques en particulier les fibres kératiniques humaines, notamment les cheveux, qui ne présentent pas les inconvénients des procédés de décoloration existants. En particulier, un des buts de la présente invention est de fournir des systèmes de coloration directe permettant d'obtenir des effets éclaircissants notamment sur des fibres kératiniques naturellement ou artificiellement foncées, tenaces face à des shampooings successifs et qui ne dégradent pas les fibres kératiniques et qui n'altèrent pas leurs propriétés cosmétiques.

Ce but est atteint avec la présente invention qui a pour objet un colorant disulfure fluorescent, pour la coloration de matières kératiniques foncées, choisi parmi les composés (V) et (X) : 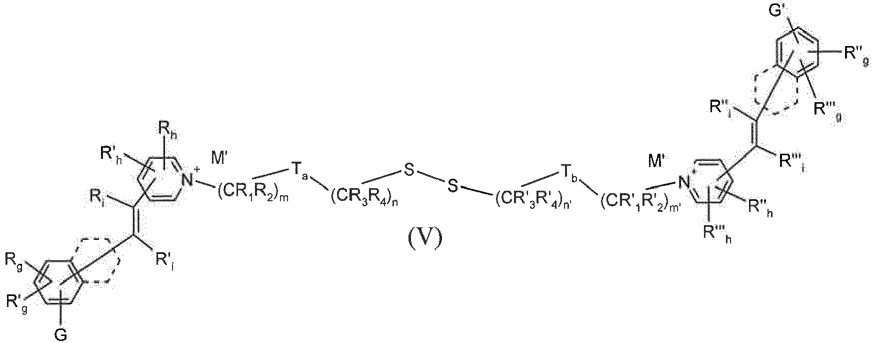 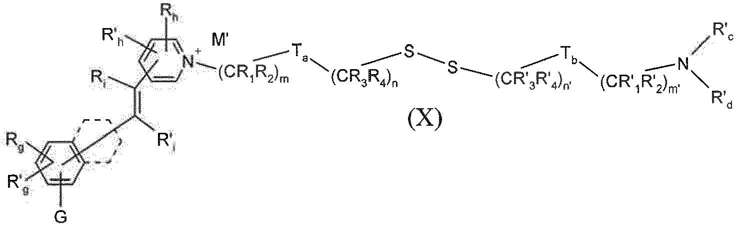 leurs sels d'acide organique ou minéral, isomères optiques, isomères géométriques, et les solvates tels que hydrates ;
dans lequel
- G et G', identiques ou différents, représentent un groupement C₁-C₆ alkoxy éventuellement substitué;
- R'_{c}, et R'_{d}, identiques ou différents, représentent un atome d'hydrogène, un groupement aryl(C₁-C₄)alkyle, C₁-C₆ alcoxy ou un groupement C₁-C₆ alkyle éventuellement substitué ;
   ou alors deux radicaux adjacents R'_{c} et R'_{d} portés par le même atome d'azote forment ensemble un groupe hétérocyclique ou hétéroaryle ;
- R_{g}, R'_{g}, R"_{g}, R"'_{g}, Rₕ, R'ₕ, R"ₕ, et R"'ₕ, identiques ou différents, représentent un atome d'hydrogène, un atome d'halogène, un groupement amino, C₁-C₄ alkylamino, C₁-C₄ dialkylamino, cyano, carboxy, hydroxyle, trifluorométhyle, un radical acylamino, alcoxy en C₁-C₄, (poly)hydroxyalcoxy en C₂-C₄, alkylcarbonyloxy alcoxycarbonyle, alkylcarbonylamino, un radical acylamino, carbamoyle , alkylsulfonylamino, un radical amino-sulfonyle, ou un radical C₁-C₁₆ alkyle éventuellement substitué par un groupement choisi parmi C₁-C₁₂ alcoxy, hydroxy, cyano, carboxy, amino, C₁-C₄ alkylamino et C₁-C₄ dialkylamino, ou alors les deux radicaux alkyles portés par l'atome d'azote du groupement amino forment un hétérocycle comprenant de 5 à 7 chaînons et comprenant éventuellement un autre hétéroatome identique ou différent à celui de l'atome d'azote ;
   ou alors deux groupements R_{g} et R'_{g}; R"_{g} et R'"_{g}; Rₕ, et R'ₕ; R"ₕ et R"'ₕ portés par deux atomes de carbone adjacents, forment ensembles une cycle benzo, indéno, un groupement hétérocycloalkyle fusionné ou hétéroaryle fusionné; le cycle benzo, indéno, hétérocycloalkyle ou hétéroaryle étant éventuellement substitué par un atome d'halogène, un groupement amino, C₁-C₄ alkylamino, C₁-C₄ dialkylamino, nitro, cyano, carboxy, hydroxyle, trifluorométhyle, un radical acylamino, alcoxy en C₁-C₄, (poly)hydroxyalcoxy en C₂-C₄, alkylcarbonyloxy alcoxycarbonyle, alkylcarbonylamino, un radical acylamino, carbamoyle, alkylsulfonylamino, un radical amino-sulfonyle, ou un radical C₁-C₁₆ alkyle éventuellement substitué par un groupement choisi parmi C₁-C₁₂ alcoxy, hydroxy, cyano, carboxy, amino, C₁-C₄ alkylamino, C₁-C₄ dialkylamino, ou alors les deux radicaux alkyles portés par l'atome d'azote du groupement amino forment un hétérocycle comprenant de 5 à 7 chaînons et comprenant éventuellement un autre hétéroatome identique ou différent à celui de l'atome d'azote ;
- Rᵢ, R'ᵢ, R"ᵢ, et R"'ᵢ, identiques ou différents, représentent un atome d'hydrogène, ou un groupement C₁-C₄ alkyle ;
- R₁, R₂, R₃, R₄, R'₁, R'₂, R'₃ et R'₄, identiques ou différents, représentent un atome d'hydrogène ou un groupement C₁-C₄ alkyle, C₁-C₁₂ alcoxy, hydroxy, cyano, carboxy, amino, C₁-C₄ alkylamino, C₁-C₄ dialkylamino, lesdits radicaux alkyles pouvant former avec l'atome d'azote qui les portent un hétérocyle comprenant de 5 à 7 chaînons, comprenant éventuellement un autre hétéroatome différent ou non de l'azote ;
- Tₐ et T_{b}, identiques ou différents, représentent i) soit une liaison covalent σ, ii) soit un ou plusieurs radicaux ou leurs combinaisons choisis parmi -SO₂-, -O-, -S-, -N(R)-, -N⁺(R)(R°)-, -CO-, avec R, R°, identiques ou différents, représentant un atome d'hydrogène, un radical alkyle en C₁-C₄, hydroxyalkyle en C₁-C₄; ou un aryl(C₁-C₄)alkyle, iii) soit un radical hétéocycloalkyle ou hétéroaryle, cationique ou non;
- 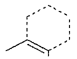 représentent un groupement aryle ou hétéroaryle fusionné au cycle phényle ; ou alors est absent du cycle phényle;
- m, m', n et n', identiques ou différents, représentent un entier compris inclusivement entre 0 et 6 avec m+n, m'+n', identiques ou différents, représente un entier compris inclusivement entre 1 et 10;
M' représentant contre-ion ou un sel d'acide organique ou minéral ;
étant entendu que le colorant de formule (V) est différent des colorants (A) et (B) suivants :

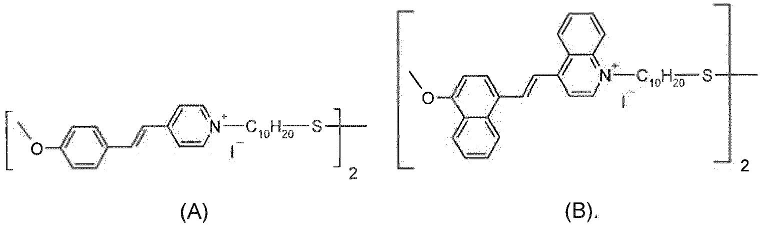

Un autre objet de l'invention est une composition tinctoriale, comprenant dans un milieu cosmétique approprié, au moins un colorant disulfure fluorescent choisi parmi les composés (V) et (X) comprenant éventuellement un agent réducteur.

Cet agent réducteur peut être choisi parmi les thiols par exemple la cystéine, l'homocystéine, l'acide thiolactique, les sels de ces thiols, les phosphines, le bisulfite, les sulfites, l'acide thioglycolique, ainsi que ses esters, notamment le monothioglycolate de glycérol, et le thioglycérol.

Cet agent réducteur peut aussi être choisi parmi les borohydrures et leurs dérivés, comme par exemple les sels du borohydrure, du cyanoborohydrure, du triacétoxyborohydrure, du triméthoxyborohydrure : sels de sodium, lithium, potassium, calcium, ammoniums quaternaires (tétraméthylammonium, tétraéthylammonium, tétra n-butylammonium, benzyltriéthylammonium); le catécholborane.

Le colorant de l'invention permet d'obtenir un éclaircissement des matières kératiniques foncées. Notamment le colorant de l'invention permet d'obtenir un éclaircissement des fibres kératiniques telle que les cheveux, très tenaces aux shampooings, aux agressions courantes (soleil, transpiration), et aux autres traitements capillaires sans dégrader la fibre kératinique.

Au sens de l'invention, on entend par matière kératinique foncée celle qui présente une luminescence L* chiffrée dans le système C.I.E. L*a*b*, inférieure ou égale à 45 et de préférence inférieure ou égale à 40, sachant par ailleurs que L*=0 équivaut au noir et L*=100 au blanc.

Au sens de l'invention, on entend par cheveux naturellement ou artificiellement foncés, des cheveux dont la hauteur de ton est inférieure ou égale à 6 (blond foncé) et de préférence inférieure ou égale à 4 (châtain).

L'éclaircissement des cheveux est évalué par la « hauteur de ton » qui caractérise le degré ou le niveau d'éclaircissement. La notion « ton » repose sur la classification des nuances naturelles, un ton séparant chaque nuance de celle qui la suit ou la précède immédiatement. Cette définition et la classification des nuances naturelles est bien connue des professionnels de la coiffure et publiée dans l'ouvrage « Science des traitements capillaires » de Charles ZVIAK 1988, Ed. Masson, p. 215 et 278.

Les hauteurs de ton s'échelonnent de 1 (noir) à 10 (blond clair clair), une unité correspondant à un ton ; plus le chiffre est élevé et plus la nuance est claire.

Un cheveu coloré artificiellement est un cheveu dont la couleur a été modifiée par un traitement de coloration par exemple une coloration avec des colorants directs ou des colorants d'oxydation.

De préférence, la composition doit, après application sur des cheveux, par exemple châtains, amener aux résultats ci-dessous.
- On s'intéresse aux performances de réflectance des cheveux lorsqu'on les irradie avec de la lumière visible dans la gamme de longueurs d'onde allant de 400 à 700 nanomètres.
- On compare alors les courbes de réflectance en fonction de la longueur d'onde, des cheveux traités avec la composition de l'invention et des cheveux non traités.
- La courbe correspondant aux cheveux traités doit montrer une réflectance dans la gamme des longueurs d'onde allant de 450 à 700 nanomètres supérieure à la courbe correspondant aux cheveux non traités.
- Cela signifie que, dans la gamme de longueur d'onde allant de 450 à 700 nanomètres, il existe au moins une plage où la courbe de réflectance correspondant aux cheveux traités est supérieure à la courbe de réflectance correspondant aux cheveux non traités. On entend par "supérieure", un écart d'au moins 0,05% de réflectance, et de préférence d'au moins 0,1%. Ceci n'empêche pas qu'il peut exister dans la gamme de longueur d'onde allant de 450 à 700 nanomètres, au moins une plage où la courbe de réflectance correspondant aux cheveux traités soit superposable, ou inférieure à la courbe de réflectance correspondant aux cheveux non traités.

De préférence, la longueur d'onde où l'écart est maximal entre la courbe de réflectance des cheveux traités et celle des cheveux non traités, se situe dans la gamme de longueur d'onde allant de 450 à 650 nanomètres, et de préférence dans la gamme de longueur d'onde allant de 450 à 620 nanomètres.

Au sens de la présente invention, et à moins qu'une indication différente ne soit donnée :
- un colorant disulfure fluorescent est un composé fluorescent comportant au moins un chromophore fluorescent tel que défini ci après, et comprenant une ou plusieurs liaisons disulfures S-S entre deux atomes de carbones, directement ou indirectement reliées au(x) chromophore(s) fluorescent(s) du composé, préférentiellement la liaison est susceptible d'être réduite dans un milieu cosmétiquement acceptable ;
- les radicaux « aryles » ou « hétéroaryles » ou la partie aryle ou hétéroaryle d'un radical peuvent être substitués par au moins un substituant porté par un atome de carbone, choisi parmi :
   - un radical alkyle en C₁-C₁₆, de préférence en C₁-C₈, éventuellement substitué par un ou plusieurs radicaux choisis parmi les radicaux hydroxy, alcoxy en C₁-C₂, (poly)-hydroxyalcoxy en C₂-C₄, acylamino, amino substitué par deux radicaux alkyle, identiques ou différents, en C₁-C₄, éventuellement porteurs d'au moins un groupement hydroxyle ou, les deux radicaux pouvant former avec l'atome d'azote auquel ils sont rattachés, un hétérocycle comprenant de 5 à 7 chaînons, de préférence de 5 ou 6 chaînons, saturé ou insaturé éventuellement substitué comprenant éventuellement un autre hétéroatome identique ou différent de l'azote ;
   - un atome d'halogène tel que chlore, fluor ou brome ;
   - un groupement hydroxyle ;
   - un radical alcoxy en C₁-C₂ ; un radical (poly)-hydroxyalcoxy en C₂-C₄ ;
   - un radical amino ;
   - un radical héterocycloalkyle à 5 ou 6 chaînons ;
   - un radical hétéroaryle à 5 ou 6 chaînons éventuellement cationique, préférentiellement imidazolium, et éventuellement substitué par un radical (C₁-C₄) alkyle, préférentiellement méthyle ;
   - un radical amino substitué par un ou deux radicaux alkyle, identiques ou différents, en C₁-C₆ éventuellement porteurs d'au moins :
      i) un groupement hydroxyle,
      ii) un groupement amino éventuellement substitué par un ou deux radicaux alkyle en C₁-C₃ éventuellement substitués, lesdits radicaux alkyle pouvant former avec l'atome d'azote auquel ils sont rattachés, un hétérocycle comprenant de 5 à 7 chaînons, saturé ou insaturé éventuellement substitué comprenant éventuellement au moins un autre hétéroatome différent ou non de l'azote,
      iii) un groupement ammonium quaternaire -N⁺R'R"R"', M⁻ pour lequel R', R", R"', identiques ou différents représentent un atome d'hydrogène, ou un groupement alkyle en C₁-C₄; et M⁻ représente le contre-ion de l'acide organique, minéral ou de l'halogénure correspondant,
      iv) ou un radical hétéroaryle à 5 ou 6 chaînons éventuellement cationique, préférentiellement imidazolium, et éventuellement substitué par un radical (C₁-C₄) alkyle, préférentiellement méthyle ;
   - un radical acylamino (-NR-COR') dans lequel le radical R est un atome d'hydrogène, un radical alkyle en C₁-C₄ éventuellement porteur d'au moins un groupement hydroxyle et le radical R' est un radical alkyle en C₁-C₂ ; un radical carbamoyle ((R)₂N-CO-) dans lequel les radicaux R, identiques ou non, représentent un atome d'hydrogène, un radical alkyle en C₁-C₄ éventuellement porteur d'au moins un groupement hydroxyle ; un radical alkylsulfonylamino (R'SO₂-NR-) dans lequel le radical R représente un atome d'hydrogène, un radical alkyle en C₁-C₄ éventuellement porteur d'au moins un groupement hydroxyle et le radical R' représente un radical alkyle en C₁-C₄, un radical phényle ; un radical aminosulfonyle ((R)₂N-SO₂-) dans lequel les radicaux R, identiques ou non, représentent un atome d'hydrogène, un radical alkyle en C₁-C₄ éventuellement porteur d'au moins un groupement hydroxyle,
   - un radical carboxylique sous forme acide ou salifiée (de préférence avec un métal alcalin ou un ammonium, substitué ou non) ;
   - un groupement cyano (CN) ;
   - un groupement polyhalogénoalkyle, préférentiellement le trifluorométhyle (CF₃) ;
- la partie cyclique ou hétérocyclique d'un radical non aromatique peut être substituée par au moins un substituant porté par un atome de carbone choisi parmi les groupements :
   - hydroxyle,
   - alcoxy en C₁-C₄, (poly)hydroxyalcoxy en C₂-C₄,
   - alkylcarbonylamino ((RCO-NR'-) dans lequel le radical R' est un atome d'hydrogène, un radical alkyle en C₁-C₄ éventuellement porteur d'au moins un groupement hydroxyle et le radical R est un radical alkyle en C₁-C₂, amino substitué par deux groupements alkyle identiques ou différents en C₁-C₄ éventuellement porteurs d'au moins un groupement hydroxyle, lesdits radicaux alkyle pouvant former avec l'atome d'azote auquel ils sont rattachés, un hétérocycle comprenant de 5 à 7 chaînons, saturé ou insaturé éventuellement substitué comprenant éventuellement au moins un autre hétéroatome différent ou non de l'azote ;
   - alkylcarbonyloxy ((RCO-O-) dans lequel le radical R est un radical alkyle en C₁-C₄, amino substitué par deux groupements alkyle identiques ou différents en C₁-C₄ éventuellement porteurs d'au moins un groupement hydroxyle, lesdits radicaux alkyle pouvant former avec l'atome d'azote auquel ils sont rattachés, un hétérocycle comprenant de 5 à 7 chaînons, saturé ou insaturé éventuellement substitué comprenant éventuellement au moins un autre hétéroatome différent ou non de l'azote ;
   - alkcoxycarbonyle ((RO-CO-) dans lequel le radical R est un radical alkyle en C₁-C₄, amino substitué par deux groupements alkyle identiques ou différents en C₁-C₄ éventuellement porteurs d'au moins un groupement hydroxyle, lesdits radicaux alkyle pouvant former avec l'atome d'azote auquel ils sont rattachés, un hétérocycle comprenant de 5 à 7 chaînons, saturé ou insaturé éventuellement substitué comprenant éventuellement au moins un autre hétéroatome différent ou non de l'azote ;
- un radical cyclique, hétérocyclique, ou une partie non aromatique d'un radical aryle ou hétéroaryle, peut également être substitué par un ou plusieurs groupements oxo ;
- une chaîne hydrocarbonée est insaturée lorsqu'elle comporte une ou plusieurs liaisons doubles et/ou une ou plusieurs liaisons triples ;
- un radical « aryle » représente un groupement mono ou polycyclique, condensé ou non, comprenant de 6 à 22 atomes de carbones, et dont au moins un cycle est aromatique ; préférentiellement le radical aryle est un phényle, biphényle, naphtyle, indényle, anthracényle, ou tétrahydronaphtyle ;
- un « radical hétéroaryle » représente un groupement mono ou polycyclique, condensé ou non, éventuellement cationique, comprenant de 5 à 22 chaînons, de 1 à 6 hétéroatomes choisis parmi l'atome d'azote, d'oxygène, de soufre et de sélénium, et dont au moins un cycle est aromatique ; préférentiellement un radical hétéroaryle est choisis parmi acridinyle, benzimidazolyle, benzobistriazolyle, benzopyrazolyle, benzopyridazinyle, benzoquinolyle, benzothiazolyle, benzotriazolyle, benzoxazolyle, pyridinyle, tetrazolyle, dihydrothiazolyle, imidazopyridinyle, imidazolyle, indolyle, isoquinolyle, naphthoimidazolyle, naphthooxazolyle, naphthopyrazolyle, oxadiazolyle, oxazolyle, oxazolopyridyle, phénazinyle, phénooxazolyle, pyrazinyle, pyrazolyle, pyrilyle, pyrazoyltriazyle, pyridyle, pyridinoimidazolyle, pyrrolyle, quinolyle, tétrazolyle, thiadiazolyle, thiazolyle, thiazolopyridinyle, thiazoylimidazolyle, thiopyrylyle, triazolyle, xanthylyle et son sel d'ammonium ;
- un « radical cyclique » est un radical cycloalkyle non aromatique, mono ou polycyclique, condensé ou non, contenant de 5 à 22 atomes de carbone, pouvant comporter de 1 à plusieurs insaturations ;
- un « radical hétérocyclique » est un radical non aromatique mono ou polycyclique, condensé ou non, contenant de 5 à 22 chaînons, comportant de 1 à 6 hétéroatomes choisis parmi l'atome d'azote, d'oxygène, de soufre et de sélénium ;
- un « radical alkyle » est un radical hydrocarboné en C₁-C₁₆, linéaire ou ramifié, de préférence en C₁-C₈,
- l'expression « éventuellement substitué » attribué au radical alkyle sous entend que ledit radical alkyle peut être substitué par un ou plusieurs radicaux choisis parmi les radicaux i) hydroxy, ii) alcoxy en C₁-C₄, iii) acylamino, iv) amino éventuellement substitué par un ou deux radicaux alkyle, identiques ou différents, en C₁-C₄, lesdits radicaux alkyles pouvant former avec l'atome d'azote qui les portent un hétérocyle comprenant de 5 à 7 chaînons, comprenant éventuellement un autre hétéroatome + différent ou non de l'azote ; v) ou un groupement ammonium quaternaire -N R'R"R"', M pour lequel R', R", R"', identiques ou différents représentent un atome + d'hydrogène, ou un groupement alkyle en C₁-C₄, ou alors -N R'R"R'" forme un hétéroaryle tel que imidazolium éventuellement substitué par un groupement C₁-C₄ alkyle, et M représente le contre-ion de l'acide organique, minéral ou de l'halogénure correspondant,
- un « radical alcoxy » est un radical alkyl-oxy pour lequel le radical alkyle est un radical hydrocarboné, linéaire ou ramifié, en C₁-C₁₆ préférentiellement en C₁-C₈ ; lorsque le groupement alcoxy est éventuellement substitué, cela sous entend que le groupe alkyle est éventuellement substitué tel que défini supra.

De plus, sauf indication contraire, les bornes délimitant l'étendue d'une plage de valeurs sont comprises dans cette plage de valeurs.

Selon la présente invention, on entend par « chromophore fluorescent» un radical issu d'un composé fluorescent. Un composé fluorescent est un composé qui est capable d'absorber dans le rayonnement UV ou visible à une longueur d'onde λ_{abs} comprise entre 250 et 800 nm et capable de réémettre dans le domaine du visible à une longueur d'onde d'émission λ_{ém} comprise entre 400 et 800 nm.

De préférence les composés fluorescents sont des colorants capables d'absorber dans le visible λ_{abs} comprise entre 400 et 800 nm et de réémettre dans le visible λ_{ém} comprise entre 400 et 800 nm. Plus préférentiellement les colorants fluorescents sont des colorants capables d'absorber à une λ_{abs} comprise entre 420 nm et 550 nm et de réémettre dans le visible à une λ_{ém} comprise entre 470 et 600 nm.

### I. Colorants de formules (V) et (X)

Plus préférentiellement, le colorant disulfure est un colorant fluorescent choisi parmi : 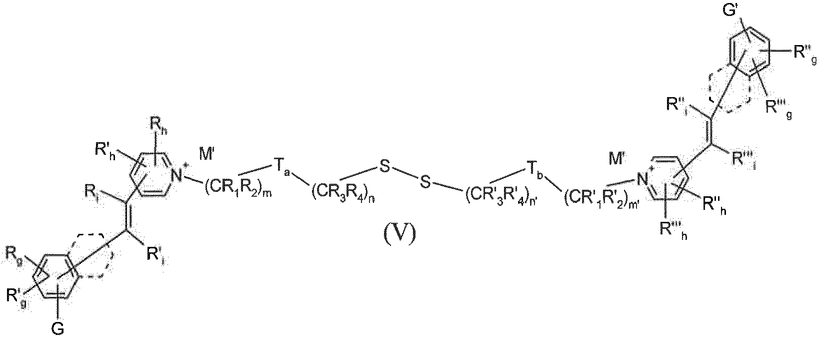 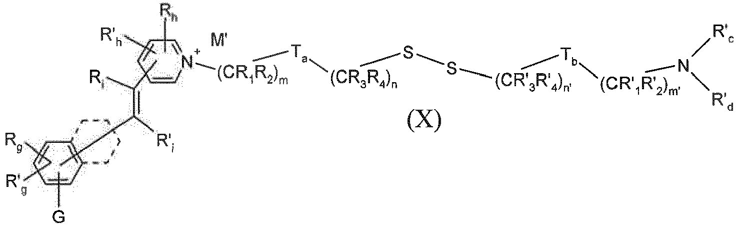 dans lequel
- G et G', identiques ou différents, représentent un groupement C₁-C₆ alkoxy éventuellement substitué, préférentiellement non substitué ;
- R'_{c}, et R'_{d}, identiques ou différents, représentent un atome d'hydrogène, un groupement aryl(C₁-C₄)alkyle, C₁-C₆ alcoxy ou un groupement C₁-C₆ alkyle éventuellement substitué ; R'_{c}, et R'_{d} représentent préférentiellement un atome d'hydrogène, un groupement hydroxy, C₁-C₃ alcoxy, amino, C₁-C₃ (di)alkylamino, ou un groupement C₁-C₃ alkyle éventuellement substitué par i) un groupement hydroxy, ii) amino, iii) C₁-C₃ (di)alkylamino, ou iv) ammonium quaternaire (R")(R"')(R"")N⁺-;
   ou alors deux radicaux adjacents R'_{c} et R'_{d} portés par le même atome d'azote forment ensemble un groupe hétérocyclique ou hétéroaryle ; préférentiellement l'hétérocycle ou l'hétéroaryle est monocyclique et comprend entre 5 et 7 chaînons ; plus préférentiellement les groupements sont choisis parmi l'imidazolyle et le pyrrolidinyle ;
- R_{g}, R'_{g}, R"_{g}, R"'_{g}, Rₕ, R'ₕ, R"ₕ, et R"'ₕ, identiques ou différents, représentent un atome d'hydrogène, un atome d'halogène, un groupement amino, C₁-C₄ alkylamino, C₁-C₄ dialkylamino, cyano, carboxy, hydroxyle, trifluorométhyle, un radical acylamino, alcoxy en C₁-C₄, (poly)hydroxyalcoxy en C₂-C₄, alkylcarbonyloxy alcoxycarbonyle, alkylcarbonylamino, un radical acylamino, carbamoyle , alkylsulfonylamino, un radical amino-sulfonyle, ou un radical C₁-C₁₆ alkyle éventuellement substitué par un groupement choisi parmi C₁-C₁₂ alcoxy, hydroxy, cyano, carboxy, amino, C₁-C₄ alkylamino et C₁-C₄ dialkylamino, ou alors les deux radicaux alkyles portés par l'atome d'azote du groupement amino forment un hétérocycle comprenant de 5 à 7 chaînons et comprenant éventuellement un autre hétéroatome identique ou différent à celui de l'atome d'azote ; préférentiellement R_{g}, R'_{g}, R"_{g}, R'"_{g}. Rₕ, R'ₕ, R"ₕ, et R"'ₕ représentent un atome d'hydrogène, d'halogène ou un groupement C₁-C₃ alkyle ;
- ou alors deux groupements R_{g} et R'_{g}; R"_{g} et R'"_{g}; Rₕ, et R'ₕ; R"ₕ et R"'ₕ portés par deux atomes de carbone adjacents, forment ensembles une cycle benzo, indéno, un groupement hétérocycloalkyle fusionné ou hétéroaryle fusionné; le cycle benzo, indéno, hétérocycloalkyle ou hétéroaryle étant éventuellement substitué par un atome d'halogène, un groupement amino, C₁-C₄ alkylamino, C₁-C₄ dialkylamino, nitro, cyano, carboxy, hydroxyle, trifluorométhyle, un radical acylamino, alcoxy en C₁-C₄, (poly)hydroxyalcoxy en C₂-C₄, alkylcarbonyloxy alcoxycarbonyle, alkylcarbonylamino, un radical acylamino, carbamoyle , alkylsulfonylamino, un radical amino-sulfonyle, ou un radical C₁-C₁₆ alkyle éventuellement substitué par : un groupement choisi parmi C₁-C₁₂ alcoxy, hydroxy, cyano, carboxy, amino, C₁-C₄ alkylamino, C₁-C₄ dialkylamino, ou alors les deux radicaux alkyles portés par l'atome d'azote du groupement amino forment un hétérocycle comprenant de 5 à 7 chaînons et comprenant éventuellement un autre hétéroatome identique ou différent à celui de l'atome d'azote; préférentiellement R_{g} et R'_{g}; R"_{g} et R'"_{g} forment ensemble un groupement benzo ;
- Rᵢ, R'ᵢ, R"ᵢ, et R"'ᵢ, identiques ou différents, représentent un atome d'hydrogène, ou un groupement C₁-C₄ alkyle ;
- R₁, R₂, R₃, R₄, R'₁, R'₂, R'₃, et R'₄, identiques ou différents, représentent un atome d'hydrogène ou un groupement C₁-C₄ alkyle, C₁-C₁₂ alcoxy, hydroxy, cyano, carboxy, amino, C₁-C₄ alkylamino, C₁-C₄ dialkylamino, lesdits radicaux alkyles pouvant former avec l'atome d'azote qui les portent un hétérocyle comprenant de 5 à 7 chaînons, comprenant éventuellement un autre hétéroatome différent ou non de l'azote ; préférentiellement R₁, R₂, R₃, R₄, R'₁, R'₂, R'₃, et R'₄ sont des atomes d'hydrogène ou un groupement amino ; plus préférentiellement R₁, R₂, R₃, R₄, R'₁, R'₂, R'₃, et R'₄ représentent un atome d'hydrogène;
- Tₐ, T_{b}, identiques ou différents, représentent i) soit une liaison covalente σ, ii) soit un ou plusieurs radicaux ou leurs combinaisons choisis parmi -SO₂- -O-, -S-, -N(R)-, -N⁺(R)(R°)-, -CO-, avec R, R°, identiques ou différents, représentant un atome d'hydrogène, un radical alkyle en C₁-C₄, hydroxyalkyle en C₁-C₄; ou un aryl(C₁-C₄)alkyle, préférentiellement Tₐ est identique à T_{b} et représentent une liaison covalente σ ou un groupement choisi parmi -N(R)-, -C(O)-N(R)-,-N(R)-C(O)-, -O-C(O)-, -C(O)-O- et -N⁺(R)(R°)-, avec R, R° identiques ou différents représentant un atome d'hydrogène ou un groupement C₁-C₄ alkyle ; plus préférentiellement Tₐ et T_{b} représentent une liaison σ ; iii) soit un radical hétérocycloalkyle ou hétéroaryle, cationique ou non, préférentiellement monocycliques, préférentiellement identiques, contenant préférentiellement deux hétéroatomes (plus préférentiellement deux atomes d'azote) et comportant préférentiellement de 5 à 7 chaînons tel que l'imidazolium;
- 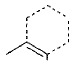 représentent un groupement aryle ou hétéroaryle fusionné au cycle phényle ; ou alors est absent du cycle phényle; préférentiellement lorsque le cycle est présent le cycle est un benzo ;
- m, m', n et n', identiques ou différents, représentent un entier compris inclusivement entre 0 et 6 avec m+n, m'+n', identiques ou différents, représente un entier compris inclusivement entre 1 et 10; préférentiellement m+n=m'+n'=un entier compris inclusivement entre 2 et 4 ; plus préférentiellement m+n=m'+n'= un entier égal à 2;
M' représentant contre-ion ou un sel d'acide organique ou minéral.

A titre d'exemple de colorants disulfures fluorescents, on peut citer notamment les composés suivants : 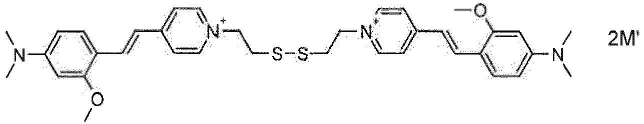 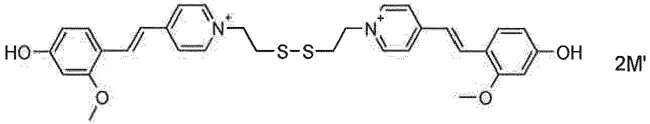 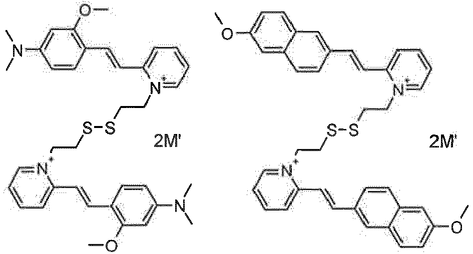 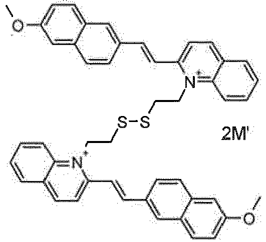 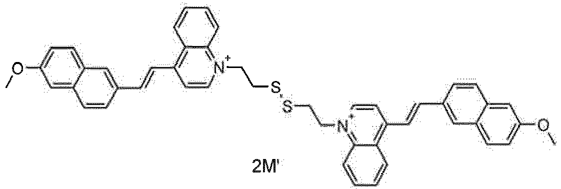 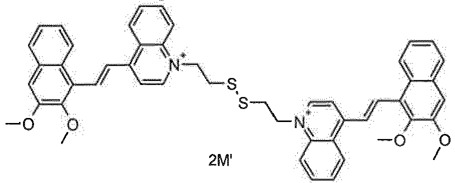 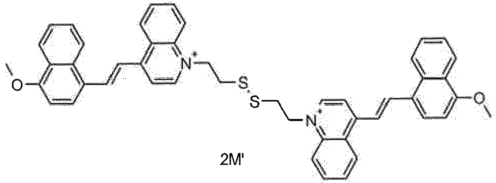 et 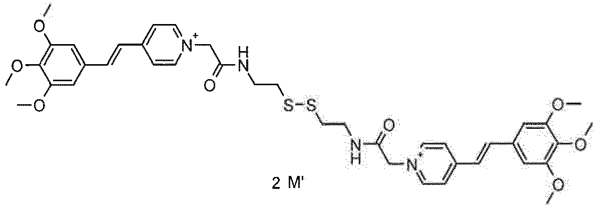 avec M' un contre-ion anionique .

### I.5. Le sel organique et contre-ion

Un « sel d'acide organique ou minéral » est plus particulièrement choisi parmi un sel dérivé i) d'acide chlorhydrique HCl, ii) d'acide bromhydrique HBr, iii) d'acide sulfurique H₂SO₄, iv) d'acides alkylsulfoniques : Alk-S(O)₂OH tels que d'acide méthylsulfonique et d'acide éthylsulfonique ; v) d'acides arylsulfoniques : Ar-S(O)₂OH tel que d'acide benzène sulfonique et d'acide toluène sulfonique ; vi) d'acide citrique ; vii) d'acide succinique ; viii) d'acide tartrique ; ix) d'acide lactique, x) d'acides alcoxysulfiniques : Alk-O-S(O)OH tels que d'acide méthoxysulfinique et d'acide éthoxysulfinique ; xi) d'acides aryloxysulfiniques tels que d'acide toluèneoxysulfinique et d'acide phénoxysulfinique ; xii) d'acide phosphorique H₃PO₄; xiii) d'acide acétique CH₃COOH ; xiv) d'acide triflique CF₃SO₃H et xv) d'acide tétrafluoroborique HBF₄.

Un « contre-ion anionique » est un anion ou un groupement anionique associé à la charge cationique du colorant ; plus particulièrement le contre-ion anionique est choisi parmi i) les halogénures tels que le chlorure, le bromure ; ii) les nitrates ; iii) les sulfonates parmi lesquels les C₁-C₆ alkylsulfonates : Alk-S(O)₂O⁻ tels que le méthylsulfonate ou mésylate et l'éthylsulfonate ; iv) les arylsulfonates : ArS(O)₂O⁻ tel que le benzènesulfonate et le toluènesulfonate ou tosylate ; v) le citrate ; vi) le succinate ; vii) le tartrate ; viii) le lactate ; ix) les alkylsulfites : Alk-O-S(O)O⁻ tels que le méthysulfite et l'éthylsulfite ; x) les arylsulfites : Ar-O-S(O)O⁻ tels que le benzènesulfite et le toluènesulfite ; xi) les alkylsulfates : Alk-O-S(O)₂O⁻ tel que le méthyl sulfate et l'éthylsulfate ; xii) les arylsulfates : Ar-O-S(O)₂O⁻, xiii) le phosphate ; xiv) l'acétate ; xv) le triflate ; et xvi) les borates tels que le tétrafluoroborate.

### I.6. Préparation des colorants disulfures fluorescents :

Les colorants disulfures fluorescents peuvent être préparés selon des méthodes connues de l'homme de l'art.

Selon une première possibilité, on peut faire réagir un composé disulfure comprenant deux fonctions amine, de préférence primaire ou secondaire avec une quantité suffisante d'un "chromophore fluorescent réactif" ou d'un composé comprenant un tel "chromophore fluorescent réactif", en d'autres termes comprenant une fonction électrophile.

Parmi les "chromophores fluorescent réactifs", on peut citer les colorants réactifs comportant notamment une fonction vinylsulfone, sulfatoéthylsulfone, mono- di-chlorotriazine, mono- di-chloro pyrimidine, difluoro chloro pyrimidine, dichloroquinoxaline, bromo-vinylsulfone.

Conviennent aussi, en tant que chromophores réactifs, les composés chromophores fluorescents comprenant au moins un groupement susceptible de réagir avec une fonction amine pour donner un groupement sulfamide (-SO₂-NR-), amide (-CO-NR-). Par exemple, on peut mentionner les groupes -SO₃W', -COOW' (avec W' représentant un atome d'hydrogène, un métal alcalin, comme le sodium, le potassium, un groupement ammonium, un groupement ammonium substitué par un ou plusieurs groupement alkyle, identiques ou non, linéaires ou ramifiés, en C₁-C₁₀, éventuellement porteurs d'au moins un hydroxyle), que l'on peut activer préalablement, selon des méthodes connues, respectivement en groupement -SO₂Cl, -COCl.

Il est ainsi envisageable de mettre en oeuvre, à titre de chromophore fluorescent réactif, les colorants acides du Colour Index répertoriés comme tels.

On pourra se référer notamment à l'ouvrage Advanced Organic Chemistry, March, 4ème Ed, pour avoir plus de détails sur les conditions opératoires mises en oeuvre.

Toujours dans le cadre de cette première possibilité, on peut mettre en oeuvre des chromophores fluorescents comprenant un groupement labile directement lié ou non au chromophore fluorescent et susceptible d'être substitué par un groupement amine, tel que Cl, Br, F, O-alkyle (par exemple O-Me), O-aryle, O-alkylaryle (par exemple O-benzyle).

Les colorants fluorescents disulfures peuvent aussi être obtenus, dans le cadre de cette possibilité, en utilisant des chromophores possédant une fonction acrylate (-OCO-C=C-) sur laquelle on effectue une réaction d'addition.

Conformément à une autre possibilité, les colorants fluorescents disulfures peuvent être obtenus en faisant réagir un composé disulfure avec un composé portant deux fonctions acide carboxylique activées selon les méthodes classiques (par exemple réaction avec un carbodiimide ou avec le chlorure de thionyle). Le produit résultant est ensuite mis à réagir avec un chromophore fluorescent porteur d'une fonction nucléophile, par exemple de type amine primaire ou secondaire, ou de type alcool aliphatique ou aromatique comme le phénol.

Là encore, on pourra se référer à l'ouvrage Advanced Organic Chemistry, March, 4ème Ed, pour avoir plus de détails sur les conditions opératoires mises en oeuvre.

Conformément à une troisième possibilité, les colorants fluorescents disulfures peuvent être obtenus par réaction d'un composé comprenant un groupement disulfure et deux groupements hydroxyle activés préalablement en groupements partants (par exemple mésylate, tosylate) avec un chromophore fluorescent portant une fonction nucléophile, avantageusement de type amine primaire, secondaire ou tertiaire, hétéroaromatique ou non, par exemple de type pyridine, imidazole, benzimidazole.

Conformément à une quatrième possibilité, les colorants fluorescents disulfures peuvent être obtenus par oxydation ménagée de colorants portant une fonction SH.

Conformément à une cinquième possibilité, et notamment pour la préparation des composés répondant à la formule (X), les colorants fluorescents disulfures peuvent être obtenus par une variante des possibilités une, deux ou trois décrites ci-dessus, en utilisant une quantité molaire de réactif disulfure supérieure ou égale à la quantité molaire de réactif contenant le groupement chromophore.

La préparation des colorants fluorescents disulfures répondant à la formule (V) pour lesquels les chromophores sont identiques est en revanche facilitée par l'emploi d'une quantité molaire de réactif contenant le groupement fluorescent chromophore de préférence supérieure ou égale à deux fois la quantité de réactifs disulfures.

### II. Composition tinctoriale:

### II.1. Colorants :

La composition tinctoriale utile dans l'invention contient en général une quantité de colorant fluorescent disulfure comprise entre 0,001 et 50% par rapport au poids total de la composition. De préférence, cette quantité est comprise entre 0,005 et 20% en poids et encore plus préférentiellement entre 0,01 et 5% en poids par rapport au poids total de la composition.

La composition tinctoriale peut en outre contenir des colorants directs additionnels. Ces colorants directs sont par exemple choisis parmi les colorants directs nitrés benzéniques neutres, acides ou cationiques, les colorants directs azoïques neutres, acides ou cationiques, les colorants tétraazapentaméthiniques, les colorants quinoniques et en particulier anthraquinoniques neutres, acides ou cationiques, les colorants directs aziniques, les colorants directs triarylméthaniques, les colorants directs indoaminiques et les colorants directs naturels.

Parmi les colorants directs naturels, on peut citer la lawsone, la juglone, l'alizarine, la purpurine, l'acide carminique, l'acide kermésique, la purpurogalline, le protocatéchaldéhyde, l'indigo, l'isatine, la curcumine, la spinulosine, l'apigénidine. On peut également utiliser les extraits ou décoctions contenant ces colorants naturels et notamment les cataplasmes ou extraits à base de henné.

La composition tinctoriale peut contenir une ou plusieurs base d'oxydation et/ou un ou plusieurs coupleurs conventionnellement utilisés pour la teinture de fibres kératiniques.

Parmi les bases d'oxydation, on peut citer les para-phénylènediamines, les bis-phénylalkylènediamines, les para-aminophénols, les bis-para-aminophénols, les ortho-aminophénols, les bases hétérocycliques et leurs sels d'addition

Parmi ces coupleurs, on peut notamment citer les méta-phénylènediamines, les méta-aminophénols, les méta-diphénols, les coupleurs naphtaléniques, les coupleurs hétérocycliques et leur sels d'addition.

Le ou les coupleurs sont chacun généralement présents en quantité comprise entre 0,001 et 10 % en poids du poids total de la composition tinctoriale, de préférence entre 0,005 et 6 %.

La ou les bases d'oxydation présentes dans la composition tinctoriale sont en général présentes chacune en quantité comprise entre 0,001 à 10 % en poids du poids total de la composition tinctoriale, de préférence entre 0,005 et 6 % en poids.

D'une manière générale, les sels d'addition des bases d'oxydation et des coupleurs utilisables dans le cadre de l'invention sont notamment choisis parmi les sels d'addition avec un acide tels que les chlorhydrates, les bromhydrates, les sulfates, les citrates, les succinates, les tartrates, les lactates, les tosylates, les benzènesulfonates, les phosphates et les acétates et les sels d'addition avec une base telles que les hydroxydes de métal alcalin comme la soude, la potasse, l'ammoniaque, les amines ou les alcanolamines.

Le milieu approprié pour la teinture, appelé aussi support de teinture, est un milieu cosmétique généralement constitué par de l'eau ou par un mélange d'eau et d'au moins un solvant organique. A titre de solvant organique, on peut par exemple citer les alcanols inférieurs en C₁-C₄, tels que l'éthanol et l'isopropanol ; les polyols et éthers de polyols comme le 2-butoxyéthanol, le propylèneglycol, le monométhyléther de propylèneglycol, le monoéthyléther et le monométhyléther du diéthylèneglycol, ainsi que les alcools aromatiques comme l'alcool benzylique ou le phénoxyéthanol, et leurs mélanges.

Les solvants lorsqu'ils sont présents sont, de préférence présents dans des proportions de préférence comprises entre 1 et 40 % en poids environ par rapport au poids total de la composition tinctoriale, et encore plus préférentiellement entre 5 et 30 % en poids environ.

Selon une variante, l'invention contient un agent réducteur capable de réduire les liaisons disulfures. Cet agent réducteur est tel que défini précédemment.

### II.2. Adjuvants:

La composition tinctoriale peut également renfermer divers adjuvants utilisés classiquement dans les compositions pour la teinture des cheveux, tels que des agents tensioactifs anioniques, cationiques, non ioniques, amphotères, zwittérioniques ou leurs mélanges, des polymères anioniques, cationiques, non ioniques, amphotères, zwittérioniques ou leurs mélanges, des agents épaississants minéraux ou organiques, et en particulier les épaississants associatifs polymères anioniques, cationiques, non ioniques et amphotères, des agents antioxydants, des agents de pénétration, des agents séquestrants, des parfums, des tampons, des agents dispersants, des agents de conditionnement tels que par exemple des silicones volatiles ou non volatiles, modifiées ou non tels que les silicones aminés, des agents filmogènes, des céramides, des agents conservateurs, des agents opacifiants, des polymères conducteurs.

### Quantité d'adjuvants :

Les adjuvants ci dessus sont en général présents en quantité comprise pour chacun d'eux entre 0,01 et 20 % en poids par rapport au poids de la composition.

Bien entendu, l'homme de l'art veillera à choisir ce ou ces éventuels composés complémentaires de manière telle que les propriétés avantageuses attachées intrinsèquement à la composition de teinture conforme à l'invention ne soient pas, ou substantiellement pas, altérées par la ou les adjonctions envisagées.

### Le pH :

Le pH de la composition tinctoriale est généralement compris entre 3 et 14 environ, et de préférence entre 5 et 11 environ. Il peut être ajusté à la valeur désirée au moyen d'agents acidifiants ou alcalinisants habituellement utilisés en teinture des fibres kératiniques ou bien encore à l'aide de systèmes tampons classiques.

Parmi les agents acidifiants, on peut citer, à titre d'exemple, les acides minéraux ou organiques comme l'acide chlorhydrique, l'acide orthophosphorique, l'acide sulfurique, les acides carboxyliques comme l'acide acétique, l'acide tartrique, l'acide citrique, l'acide lactique, les acides sulfoniques.

Parmi les agents alcalinisants on peut citer, à titre d'exemple, l'ammoniaque, les carbonates alcalins, les alcanolamines telles que les mono-, di- et triéthanolamines ainsi que leurs dérivés, les hydroxydes de sodium ou de potassium et les composés de formule (a) suivante : 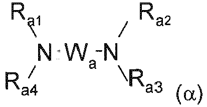 dans laquelle Wₐ est un reste propylène éventuellement substitué par un groupement hydroxyle ou un radical alkyle en C₁-C₄ ; Rₐ₁, Rₐ₂, Rₐ₃ et Rₐ₄, identiques ou différents, représentent un atome d'hydrogène, un radical alkyle en C₁-C₄ ou hydroxyalkyle en C₁-C₄.

### II.3. Formes de la composition :

La composition tinctoriale peut se présenter sous des formes diverses, telles que sous forme de liquide, de crème, de gel, ou sous toute autre forme appropriée pour réaliser une teinture des fibres kératiniques, et notamment des cheveux.

### III. Procédé de coloration:

L'application de la composition tinctoriale est généralement effectuée à température ambiante. Elle peut cependant être réalisée à des températures variant de 20 à 180°C.

Dans un procédé particulier de coloration de l'invention, l'application du colorant fluorescent disulfure sur les matières kératiniques est réalisée en même temps qu'un agent réducteur. L'agent réducteur est tel que défini précédemment.

Selon une autre variante l'agent réducteur est ajouté à la composition tinctoriale contenant les colorants fluorescents disulfures (V) et (X) au moment de l'emploi.

Suivant une autre variante le procédé de l'invention peut être mis en oeuvre en présence d'un agent d'oxydation en post traitement lorsque la composition contient déjà un agent réducteur.

L'agent oxydant peut être n'importe quel agent oxydant utilisé de façon classique dans le domaine. Ainsi, il peut être choisi parmi le peroxyde d'hydrogène, le peroxyde d'urée, les bromates de métaux alcalins, les persels tels que les perborates et les persulfates, ainsi que les enzymes parmi lesquelles on peut citer les peroxydases, les oxydo-réductases à 2 électrons telles que les uricases et les oxygénases à 4 électrons comme les laccases. L'utilisation du peroxyde d'hydrogène est particulièrement préférée.

Cet agent oxydant peut être appliqué sur les fibres avant ou après l'application de la composition contenant les colorants fluorescents disulfures de formule (V) et (X).

Les exemples qui suivent servent à illustrer l'invention sans toutefois présenter un caractère limitatif. Les colorants fluorescents disulfures des exemples ci-après ont été entièrement caractérisés par les méthodes spectroscopiques et spectrométriques classiques.

### EXEMPLES

### EXEMPLES DE SYNTHESE

### Exemple 1

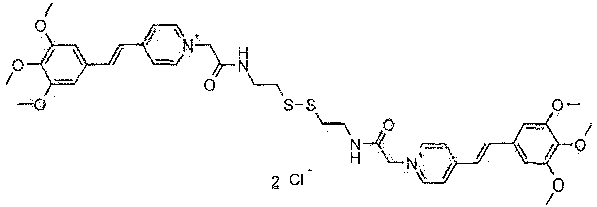

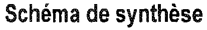

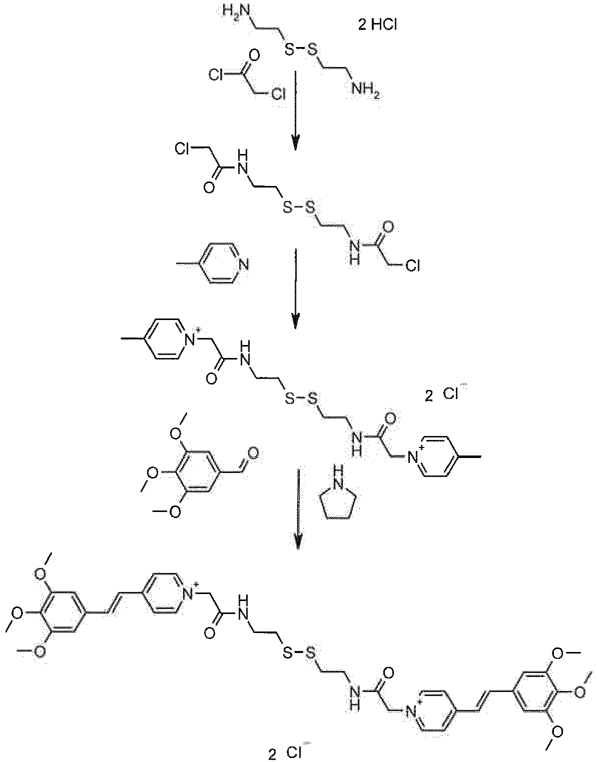

### Etape 1 : Synthèse du N,N'-(disulfanediyldiéthane-2,1-diyl)bis(2-chloroacétamide)

40,3 g de dichlorhydrate de cystamine sont dissous dans 100 mL d'eau, 32 mL de soude à 35 % sont ajoutés (pH 9.7) et la température est abaissée à 5 °C. 33,5 mL de chlorure de chloracétyle sont introduits goutte à goutte, en maintenant la température inférieure à 10 °C et en maintenant le pH entre 7,9 et 9,3 par addition de soude. Le milieu est maintenu agité à température ambiante pendant 2 H. Le précipité est filtré, lavé par 5 x 150 mL d'eau puis séché sous vide en présence de P₂O₅. 35,3 g de poudre blanche sont recueillis. les analyses indiquent que le produit est conforme.

### Etape 2 : Synthèse du dichlorure de 1,1'-{disulfanediylbis[éthane-2,1-diylimino(2-oxoéthane-2,1-diyl)]}bis(4-méthylpyridinium)

6,1 g N,N'-(disulfanediyldiéthane-2,1-diyl)bis(2-chloroacétamide) et 4,5 g de 4-picoline sont dissous dans 50 mL de NMP et portés à 80 °C pendant 19 h. Après refroidissement du mélange, par précipitations successives dans l'acétone et séchage sous vide, 9,2 g de sels sont recueillis. Les analyses montrent que le produit est conforme. RMN ¹H (400 MHz, D₂O) : 2.61 (s, 6 H), 2.82 (t, 4 H), 3.56 (t, 4 H), 5.31 (s, 4 H), 7.85 (d, 4 H), 8.51 (d, 4 H).

### Etape 3 : Synthèse du dichlorure de 1,1'-{disulfanediylbis[éthane-2,1-diylimino(2-oxoéthane-2,1-diyl)]}bis{4-[(E)-2-(3,4,5-triméthoxyphényl)vinyl]pyridinium} [4]

785 mg de 3,4,5-triméthoxy-benzaldéhyde, 328 µL de pyrrolidine, 232 µL d'acide acétique et 490 mg de dichlorure de 1,1'-{disulfanediylbis[éthane-2,1-diylimino(2-oxoéthane-2,1-diyl)]}bis(4-méthylpyridinium) sont mis en solution dans 10 mL d'isopropanol et maintenus agités à température ambiante 3 h 30 min. Le mélange est versé sur 50 mL de solution de dichlorométhane / acétone 1 :1. Un solide précipite. Il est filtré, lavé par trois fois 20 mL d'acétone et séché sous vide. 509 mg de poudre noire sont recueillis. Les analyses montrent que le produit est conforme (LCMS : 100% ; pic de masse m/z = 388, correspondant au dication).

## Revendications

1. Colorant fluorescent disulfure choisi parmi les composés (V) et (X) : 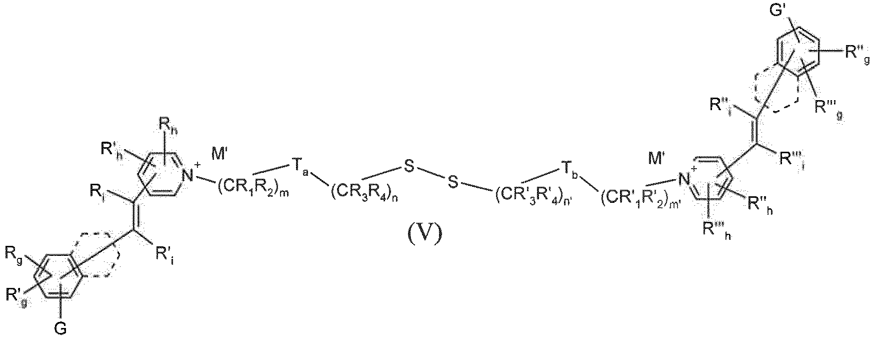 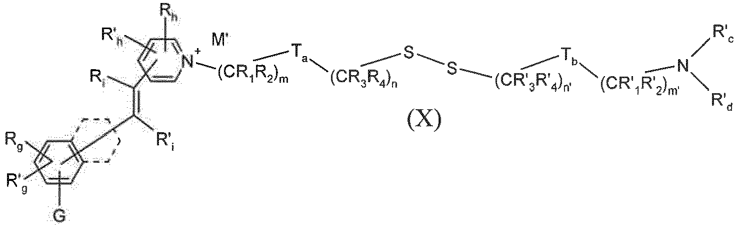 leurs sels d'acide organique ou minéral, isomères optiques, isomères géométriques, et les solvates tels que hydrates ;
dans lequel
• G et G', identiques ou différents, représentent un groupement C₁-C₆ alkoxy éventuellement substitué;
• R'_{c}, et R'_{d}, identiques ou différents, représentent un atome d'hydrogène, un groupement aryl(C₁-C₄)alkyle, C₁-C₆ alcoxy ou un groupement C₁-C₆ alkyle éventuellement substitué ;
ou alors deux radicaux adjacents R'_{c} et R'_{d} portés par le même atome d'azote forment ensemble un groupe hétérocyclique ou hétéroaryle ;
• R_{g}, R'_{g}, R"_{g}, R"'_{g}, Rₕ, R'ₕ, R"ₕ, et R"'ₕ, identiques ou différents, représentent un atome d'hydrogène, un atome d'halogène, un groupement amino, C₁-C₄ alkylamino, C₁-C₄ dialkylamino, cyano, carboxy, hydroxyle, trifluorométhyle, un radical acylamino, alcoxy en C₁-C₄, (poly)hydroxyalcoxy en C₂-C₄, alkylcarbonyloxy alcoxycarbonyle, alkylcarbonylamino, un radical acylamino, carbamoyle , alkylsulfonylamino, un radical amino-sulfonyle, ou un radical C₁-C₁₆ alkyle éventuellement substitué par un groupement choisi parmi C₁-C₁₂ alcoxy, hydroxy, cyano, carboxy, amino, C₁-C₄ alkylamino et C₁-C₄ dialkylamino, ou alors les deux radicaux alkyles portés par l'atome d'azote du groupement amino forment un hétérocycle comprenant de 5 à 7 chaînons et comprenant éventuellement un autre hétéroatome identique ou différent à celui de l'atome d'azote ;
ou alors deux groupements R_{g} et R'_{g}; R"_{g} et R"'_{g}; Rₕ, et R'ₕ ; R"ₕ et R"'ₕ portés par deux atomes de carbone adjacents, forment ensembles une cycle benzo, indéno, un groupement hétérocycloalkyle fusionné ou hétéroaryle fusionné; le cycle benzo, indéno, hétérocycloalkyle ou hétéroaryle étant éventuellement substitué par un atome d'halogène, un groupement amino, C₁-C₄ alkylamino, C₁-C₄ dialkylamino, nitro, cyano, carboxy, hydroxyle, trifluorométhyle, un radical acylamino, alcoxy en C₁-C₄, (poly)hydroxyalcoxy en C₂-C₄, alkylcarbonyloxy alcoxycarbonyle, alkylcarbonylamino, un radical acylamino, carbamoyle, alkylsulfonylamino, un radical amino-sulfonyle, ou un radical C₁-C₁₆ alkyle éventuellement substitué par un groupement choisi parmi C₁-C₁₂ alcoxy, hydroxy, cyano, carboxy, amino, C₁-C₄ alkylamino, C₁-C₄ dialkylamino, ou alors les deux radicaux alkyles portés par l'atome d'azote du groupement amino forment un hétérocycle comprenant de 5 à 7 chaînons et comprenant éventuellement un autre hétéroatome identique ou différent à celui de l'atome d'azote ;
• Rᵢ, R'ᵢ, R"ᵢ, et R"₁, identiques ou différents, représentent un atome d'hydrogène, ou un groupement C₁-C₄ alkyle ;
• R₁, R₂, R₃, R₄, R'₁, R'₂, R'₃ et R'₄, identiques ou différents, représentent un atome d'hydrogène ou un groupement C₁-C₄ alkyle, C₁-C₁₂ alcoxy, hydroxy, cyano, carboxy, amino, C₁-C₄ alkylamino, C₁-C₄ dialkylamino, lesdits radicaux alkyles pouvant former avec l'atome d'azote qui les portent un hétérocyle comprenant de 5 à 7 chaînons, comprenant éventuellement un autre hétéroatome différent ou non de l'azote ;
• Tₐ et T_{b}, identiques ou différents, représentent i) soit une liaison covalent σ, ii) soit un ou plusieurs radicaux ou leurs combinaisons choisis parmi -SO₂-, -O-, -S-, -N(R)-, -N⁺(R)(R°)-, -CO-, avec R, R°, identiques ou différents, représentant un atome d'hydrogène, un radical alkyle en C₁-C₄, hydroxyalkyle en C₁-C₄; ou un aryl(C₁-C₄)alkyle, iii) soit un radical hétérocycloalkyle ou hétéroaryle, cationique ou non;
• 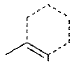 représentent un groupement aryle ou hétéroaryle fusionné au cycle phényle ; ou alors est absent du cycle phényle;
• m, m', n et n', identiques ou différents, représentent un entier compris inclusivement entre 0 et 6 avec m+n, m'+n', identiques ou différents, représente un entier compris inclusivement entre 1 et 10;
M' représentant contre-ion ou un sel d'acide organique ou minéral ; étant entendu que le colorant de formule (V) est différent des colorants (A) et (B) suivants :

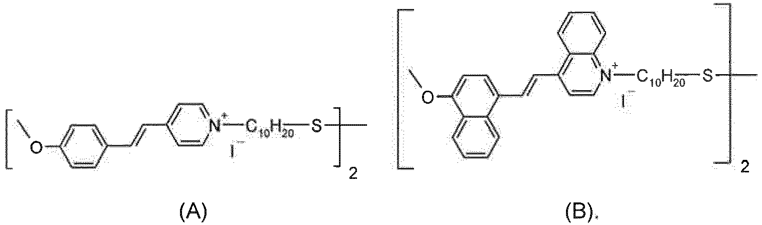

2. Colorant fluorescent disulfure selon la revendication précédente choisi parmi : 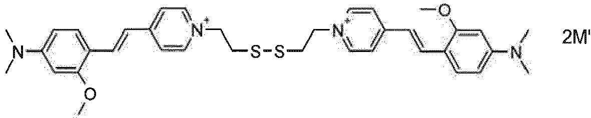 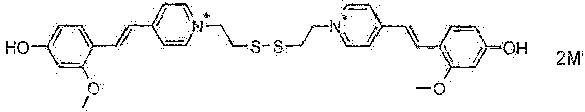 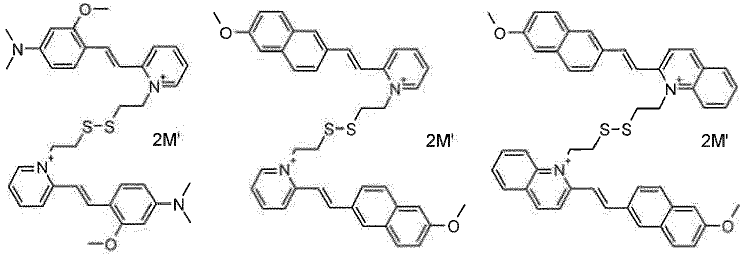 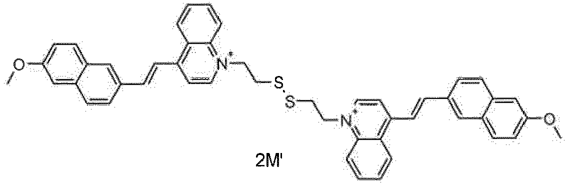 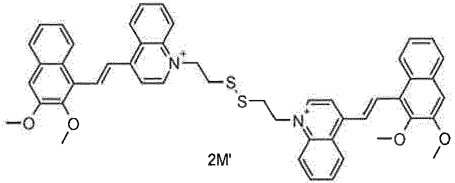 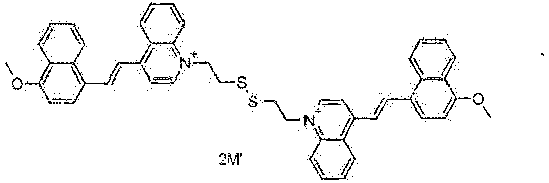 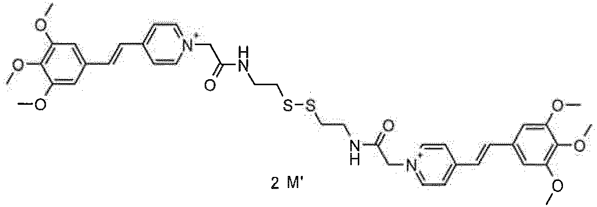 avec M' un contre-ion anionique.

3. Composition tinctoriale, comprenant dans un milieu cosmétique approprié, au moins un colorant disulfure fluorescent choisi parmi les composés (V) et (X) selon la revendication 1.

4. Composition tinctoriale, comprenant dans un milieu cosmétique approprié, au moins un colorant disulfure fluorescent selon la revendication 2.

5. Composition selon la revendication 3 ou 4, comprenant un agent réducteur.

6. Composition tinctoriale, selon la revendication précédente **caractérisé en ce que** l'agent réducteur est choisi parmi la cystéine, l'homocystéine, l'acide thiolactique, les sels de ces thiols, les phosphines, le bisulfite, les sulfites et l'acide thioglycolique, ainsi que les esters de l'acide thioglycolique.

7. Composition tinctoriale, selon une quelconque des revendications 3 à 6, dans laquelle le colorant fluorescent disulfure est présent en quantité comprise entre 0,001 et 50% en poids, de préférence, entre 0,005 et 20% en poids et encore plus préférentiellement entre 0,01 et 5% en poids, par rapport au poids total de la composition tinctoriale.

## Patentansprüche

1. Disulfid-Fluoreszenzfarbstoff, ausgewählt aus den Verbindungen (V) und (X): 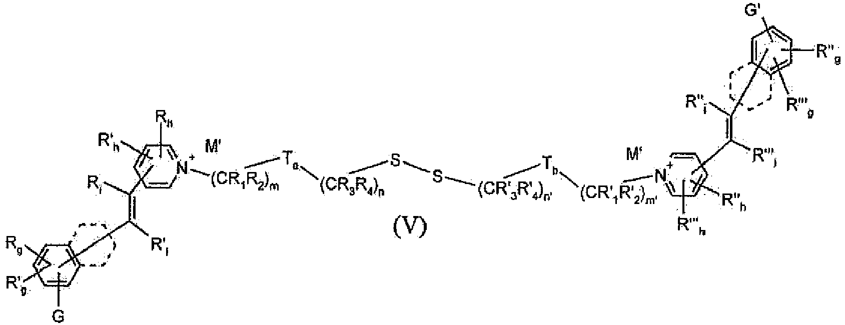 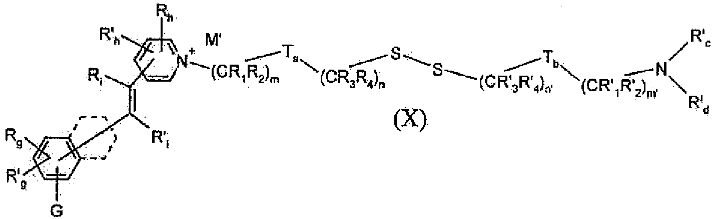 Salzen davon mit einer organischen oder anorganischen Säure, optischen Isomeren, geometrischen Isomeren und Solvaten wie Hydraten; worin
• G und G_{'} gleich oder verschieden sind und für eine gegebenenfalls substituierte C₁-C₆-Alkoxygruppe stehen;
• R'_{c} und R'_{d} gleich oder verschieden sind und für ein Wasserstoffatom, eine Aryl (C₁-C₄) alkylgruppe, eine C₁-C₆-Alkoxygruppe oder eine C₁-C₆-Alkylgruppe, die gegebenenfalls substituiert ist, stehen;
oder auch zwei benachbarte Reste R'_{c} und R'_{d}, die an dasselbe Stickstoffatom gebunden sind, zusammen eine heterocyclische Gruppe oder Heteroarylgruppe bildern;
• R_{g}, R'_{g}, R"_{g}, R"'_{g}, Rₕ, R'ₕ, R"ₕ und R'"ₕ gleich oder verschieden sind und für ein Wasserstoffatom, ein Halogenatom, eine Amino-, C₁-C₄-Alkylamino-, C₁-C₄-Dialkylamino-, Cyano-, Carboxy-, Hydroxyl- oder Trifluormethylgruppe, einen Acylamino-, C₁-C₄-Alkoxy-, (Poly)hydroxy-(C₂-C₄) alkoxy-, Alkylcarbonyloxy-, Alkoxycarbonyl- oder Alkylcarbonylaminorest, einen Acylamino-, Carbamoyl- oder Alkylsulfonylaminorest, einen Aminosulfonylrest oder einen C₁-C₁₆-Alkylrest, der gegebenenfalls durch eine aus C₁-C₁₂-Alkoxy, Hydroxy, Cyano, Carboxy, Amino, C₁-C₄-Alkylamino und C₁-C₄-Dialkylamino ausgewählte Gruppe substituiert ist, stehen oder auch die beiden Alkylreste, die an das Stickstoffatom der Aminogruppe gebunden sind, einen 5- bis 7-gliedrigen Heterocyclus bilden, der gegebenenfalls ein weiteres Heteroatom, das mit demjenigen des Stickstoffatoms identisch oder davon verschieden ist, enthält;
oder auch zwei Gruppen R_{g} und R'_{g}; R"_{g} und R"'_{g}; Rₕ und R'ₕ; R"ₕ und R"'ₕ, die an zwei beriachbarte Kohlenstoffatome gebunden sind, zusammen einen Benzo- oder Indenoring oder eine kondensierte Heterocycloalkyl- oder kondensierte Heteroarylgruppe bilden; wobei der Benzo-, Indeno-, Heterocycloalkyl- oder Heteroarylring gegebenenfalls durch ein Halogenatom, eine Amino-, C₁-C₄-Alkylamino-, C₁-C₄-Dialkylamino-, Nitro-, Cyano-, Carboxy-, Hydroxyl- oder Trifluormethylgruppe, einen Acylamino-, C₁-C₄-Alkoxy-, (Poly) hydroxy (C₂-C₄) alkoxy-, Alkylcarbonyloxy-, Alkoxycarbonyl- oder Alkylcarbonylaminorest, einen Acylamino-, Carbamoyl- oder Alkylsulfonylaminorest, einen Aminosulfonylrest oder einen C₁-C₁₆-Alkylrest, der gegebenenfalls durch eine aus C₁-C₁₂-Alkoxy, Hydroxy, Cyano, Carboxy, Amino, C₁-C₄-Alkylamino und C₁-C₄-Dialkylamino ausgewählte Gruppe substituiert ist, substituiert ist oder auch die beiden Alkylreste, die an das Stickstoffatom der Aminogruppe gebunden sind, einen 5- bis 7-gliedrigen Heterocyclus bilden, der gegebenenfalls ein weiteres Heteroatom, das mit demjenigen des Stickstoffatoms identisch oder davon verschieden ist, enthält;
• Rᵢ, R'ᵢ, R"ᵢ und R"'ᵢ gleich oder verschieden sind und für ein Wasserstoffatom oder eine C₁-C₄-Alkylgruppe stehen;
• R₁, R₂, R₃, R₄, R'₁, R'₂, R'₃ und R'₄ gleich oder verschieden sind und für ein Wasserstoffatom oder eine C₁-C₄-Alkyl-, C₁-C₁₂-Alkoxy-, Hydroxy-, Cyano-, Carboxy-, Amino-, C₁-C₄-Alkylamino- oder C₁-C₄-Dialkylaminogruppe stehen, wobei die Alkylreste mit dem Stickstoffatom, an das sie gebunden sind, einen 5- bis 7-gliedrigen Heterocyclus bilden können, der gegebenenfalls ein weiteres Heteroatom, das mit Stickstoff identisch oder davon verschieden ist, enthält;
• Tₐ und T_{b} gleich oder verschieden sind und für i) eine kovalente σ-Bindung, ii) einen oder mehrere Reste oder Kombinationen davon, die aus -SO₂-, -O-., -S-, -N(R)-, -N⁺(R)(R°)- und -CO-, wobei R und R° gleich oder verschieden sind und für ein Wasserstoffatom oder einen C₁-C₄Alkyl-, C₁-C₄-Hydroxyalkyl- oder Aryl(C₁-C₄)alkylrest stehen, ausgewählt sind, iii) oder einen kationischen oder nichtkationischen Heterocycloalkyl- oder Heteroarylrest stehen;
• 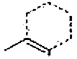 für eine mit einem Phenylring kondensierte Aryl- oder Heteroarylgruppe steht oder auch an dem Phenylring fehlt;
• m, m', n und n' gleich oder verschieden sind und für eine ganze Zahl zwischen 0 und 6 inklusive stehen, wobei m+n, m'+n' gleich oder verschieden sind und für eine ganze Zahl zwischen 1 und 10 inklusive stehen;
wobei M' für ein Gegenion oder ein Salz einer organischen oder anorganischen Säure steht;
mit der Maßgabe, dass der Farbstoff der Formel (V) von den folgenden Farbstoffen (A) und (B) verschieden ist:

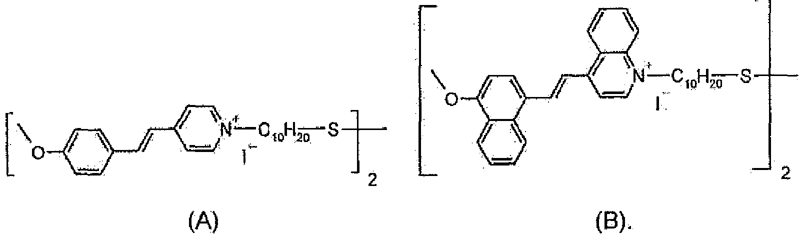

2. Disulfid-Fluoreszenzfarbstoff nach dem vorhergehenden Anspruch, ausgewählt aus: 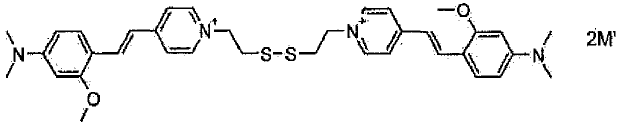 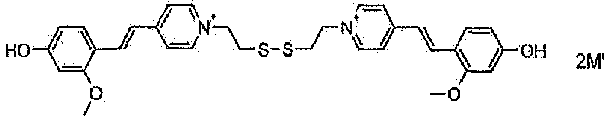 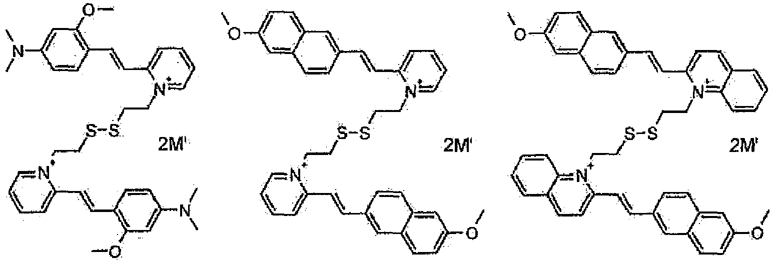 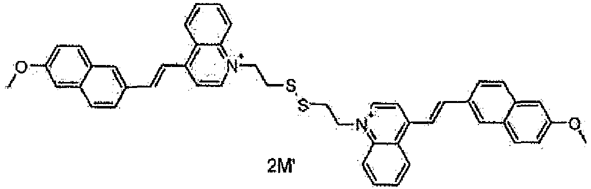 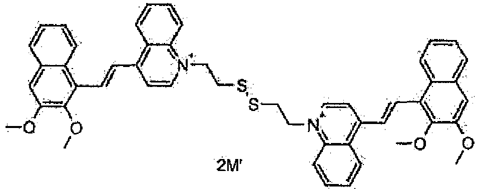 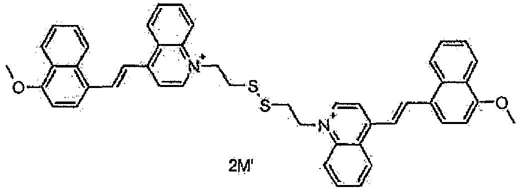 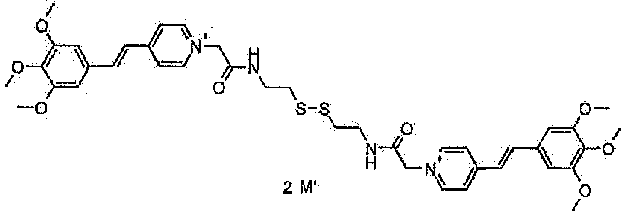 wobei M' für ein anionisches Gegenion steht.

3. Färbezusammensetzung, die in einem geeigneten kosmetischen Medium mindestens einen Disulfid-Fluoreszenzfarbstoff, der aus den Verbindungen (V) und (X) gemäß Anspruch 1 ausgewählt ist, umfasst.

4. Färbezusammensetzung, die in einem geeigneten kosmetischen Medium mindestens einen Disulfid-Fluoreszenzfarbstoff gemäß Anspruch 2 umfasst.

5. Zusammensetzung nach Anspruch 3 oder 4, die ein Reduktionsmittel umfasst.

6. Färbezusammensetzung nach dem vorhergehenden Anspruch, **dadurch gekennzeichnet, dass** das Reduktionsmittel aus Cystein, Homocystein, Thiomilchsäure, den Salzen dieser Thiole, Phosphinen, Hydrogensulfit, Sulfiten und Thioglykolsäure sowie den Estern von Thioglycolsäure ausgewählt ist.

7. Färbezusammensetzung nach einem der Ansprüche 3 bis 6, wobei der Disulfid-Fluoreszenzfarbstoff in einer Menge zwischen 0,001 und 50 Gew.-%, vorzugsweise zwischen 0,005 und 20 Gew.-% und noch weiter bevorzugt zwischen 0,01 und 5 Gew.-%, bezogen auf das Gesamtgewicht der Färbezusammensetzung, vorliegt.

## Claims

1. Fluorescent disulfide dye chosen from compounds (V) and (X) : 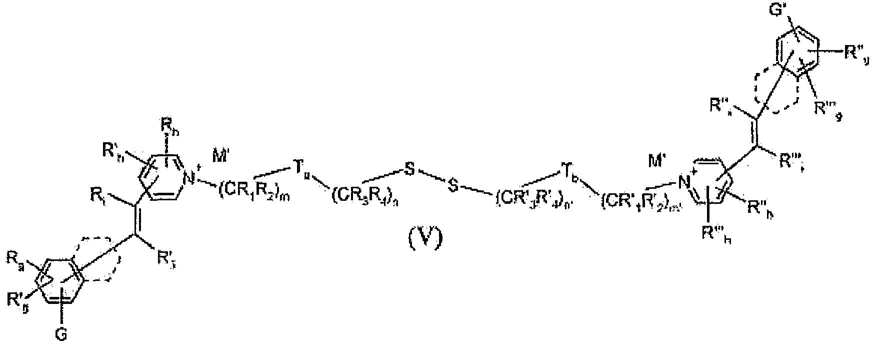 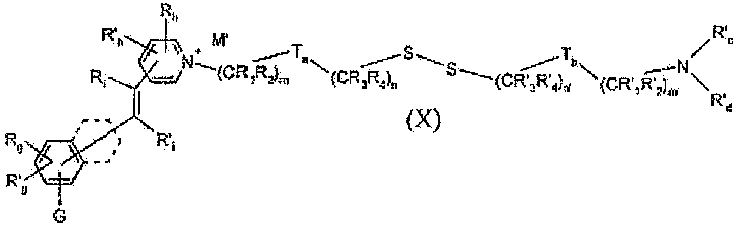 the organic or mineral acid salts, optical isomers, geometric isomers, and solvates, such as hydrates, thereof;
in which
• G and G', which are identical or different, represent an optionally substituted C₁-C₆ alkoxy group;
• R'_{c} and R'_{d}, which are identical or different, represent a hydrogen atom, an aryl(C₁-C₄)alkyl or C₁-C₆ alkoxy group or an optionally substituted C₁-C₆ alkyl group;
or else two adjacent radicals R'_{c} and R'_{d} borne by the same nitrogen atom together form a heterocyclic or heteroaryl group;
• R_{g}, R'_{g}, R"_{g}, R"'_{g}, Rₕ, R'ₕ, R"ₕ and R"'ₕ, which are identical or different, represent a hydrogen atom, a halogen atom, an amino, C₁-C₄ alkylamino, C₁-C₄ dialkylamino, cyano, carboxy, hydroxyl or trifluoromethyl group, an acylamino, C₁-C₄ alkoxy, (poly) hydroxy (C₂-C₄) alkoxy, alkylcarbonyloxy, alkoxycarbonyl or alkylcarbonylamino radical, an acylamino, carbamoyl or alkylsulfonylamino radical, an aminosulfonyl radical, or a C₁-C₁₆ alkyl radical optionally substituted with a group chosen from C₁-C₁₂ alkoxy, hydroxyl, cyano, carboxy, amino, C₁-C₄ alkylamino, and C₁-C₄ dialkylamino, or else the two alkyl radicals borne by the nitrogen atom of the amino group form a 5-to 7-membered heterocycle optionally comprising, another heteroatom identical to or different from that of the nitrogen atom;
or else two groups R_{g} and R'_{g}; R"_{g} and R"'_{g}; Rₕ and R'ₕ; R"ₕ and R"'ₕ borne by two adjacent carbon atoms together form a benzo, or inderso ring, a fused heterocycloalkyl or fused heteroaryl group; the benzo, indeno, heterocycloalkyl or heteroaryl ring being optionally substituted with a halogen atom, an amino, C₁-C₄ alkylamino, C₁-C₄ dialkylamixio, nitro, cyano, carboxy, hydroxyl or trifluoromethyl group, an acylamino, C₁-C₄ alkoxy, (poly)hydroxy(C₂-C₄)alkoxy, alkylcarbonyloxy, alkoxycarbonyl or alkylcarbonylamino radical, an acylamino, carbamoyl or alkylsulfonylamino radical, an aminosulfonyl radical, or a C₁-C₁₆ alkyl radical optionally substituted with a group chosen from C₁-C₁₂ alkoxy, hydroxyl, cyano, carboxy, amino, C₁-C₄ alkylamino, C₁-C₄ dialkylamino, or else the two alkyl radicals borne by the nitrogen atom of the amino group form a 5- to 7-membered heterocycle optionally comprising, another heteroatom identical to or different from that of the nitrogen atom;
• Rᵢ, R'ᵢ, R"ᵢ, and R"'ᵢ, which are identical or different, represent a hydrogen atom or a C₁-C₄ alkyl group;
• R₁, R₂, R₃, R₄, R'₁, R'₂, R'₃, and R'₄, which are identical or different, represent a hydrogen atom or a C₁-C₄ alkyl, C₁-C₁₂ alkoxy, hydroxyl, cyano, carboxy, amino, C₁-C₄ alkylamino or C₁-C₄ dialkylamino, group, said alkyl radicals possibly forming with the nitrogen atom that bears them a 5- to 7-membered heterocycle optionally comprising another nitrogen or non-nitrogen heteroatom;
• Tₐ and T_{b}, which are identical or different, represent i) either a covalent σ bond, ii) or one or more radicals or combinations thereof chosen from -SO₂-, -0-, -S-, -N(R)-, -N⁺(R)(R°)⁻, -CO-, with R, R°, which are identical or different, representing a hydrogen atom, a C₁-C₄ alkyl radical or a C₁-C₄ hydroxyalkyl radical; or an aryl(C₁-C₄)alkyl; iii) or a cationic or non-cationic heterocycloalkyl or heteroaryl radical,
• 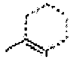 representing an aryl or heteroaryl group fused to the phenyl ring; or else is absent from the phenyl ring;
• m, m', n and n', which are identical or different, represent an integer between 0 and 6 inclusive, with m+n and m'+n', which are identical or different, representing an integer between 1 and 10 inclusive;
M' representing a counterion or an organic or mineral acid salt;
it being understood that the dye of formula (V) is different to the following dyes (A) and (B):

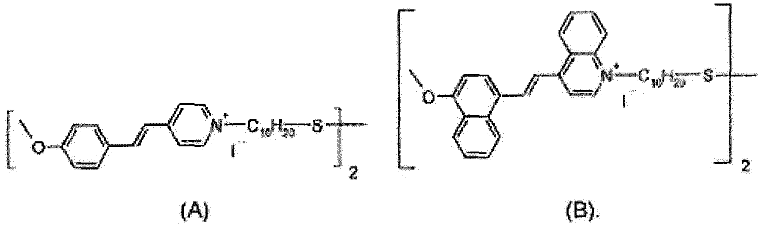

2. Fluorescent disulfide dye according to the preceding claim, chosen from: 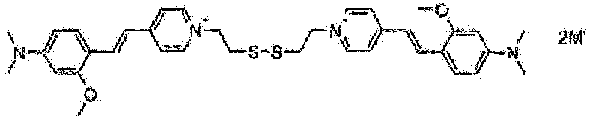 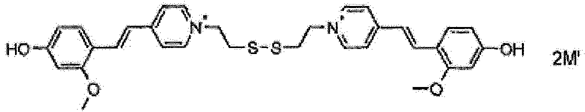 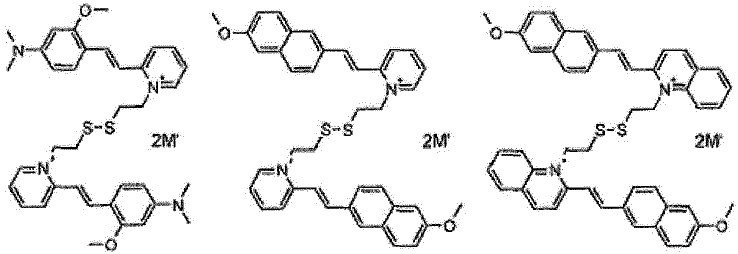 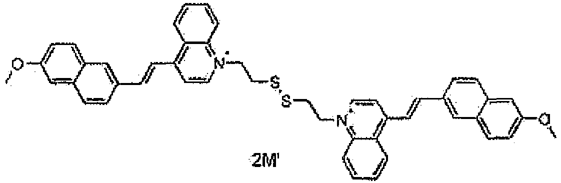 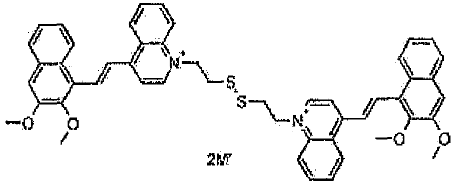 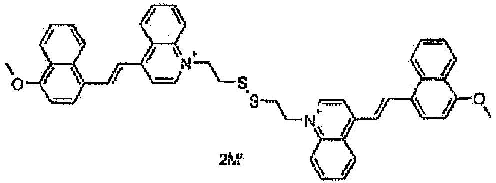 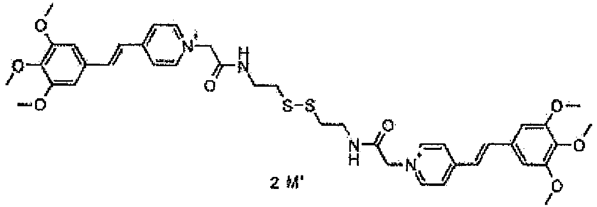 with M' being an anionic counterion.

3. Dye composition, comprising, in a suitable cosmetic medium, at least one fluorescent disulfide dye chosen from compounds (V) and (X) according to Claim 1.

4. Dye composition, comprising, in a suitable cosmetic Medium, at least one fluorescent disulfide dye according to Claim 2.

5. Composition according to Claim 3 or 4, comprising a reducing agent.

6. Dye composition according to the preceding claim, **characterized in that** the reducing agent is chosen from cysteine, homocysteine, thiolactic acid, the salts of these thiols, phosphines, bisulfite, sulfites and thioglycolic acid, and also the esters of thioglycolic acid.

7. Dye composition according to any one of Claims 3 to 6, in which the fluorescent disulfide dye is present in an amount of between 0.001% and 50% by weight, preferably between 0.005% and 20% by weight, and even more preferentially between 0.01% and 5% by weight, relative to the total weight of the dye composition.
